(19) Europäisches Patentamt
European Patent Office
Office européen des brevets

(11) **EP 4 755 293 A1**

(12) **EUROPEAN PATENT APPLICATION**

(43) Date of publication:
**10.06.2026 Bulletin 2026/24**

(21) Application number: **25220076.1**

(22) Date of filing: **02.12.2025**

(51) International Patent Classification (IPC):
**A61B 5/00** (2006.01)

(52) Cooperative Patent Classification (CPC):
**A61B 5/0071; A61B 5/0075; A61B 5/444**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **05.12.2024 IT 202400027546**

(71) Applicants:
• **Università Cattolica del Sacro Cuore**
  **20123 Milano (IT)**
• **Fondazione Policlinico Universitario Agostino Gemelli IRCCS**
  **00168 Roma (RM) (IT)**

(72) Inventors:
• **MAULUCCI, Giuseppe**
  **00168 Roma (RM) (IT)**
• **DE SPIRITO, Marco**
  **20123 Milano (MI) (IT)**
• **RIENTE, Alessia**
  **00168 Roma (RM) (IT)**
• **BIANCHETTI, Giada**
  **00168 Roma (RM) (IT)**

(74) Representative: **Manna, Sara**
  **Società Italiana Brevetti S.p.A.**
  **Piazza di Pietra, 39**
  **00186 Roma (IT)**

(54) **SYSTEM FOR DIAGNOSING THE HEALTH STATUS OF THE SKIN**

(57) The present invention relates to a portable system for diagnosing the health status of a patient's skin according to a not invasive mode, based on the analysis of the skin autofluorescence spectrum.

The system is configured to emit an excitation light radiation in the ultraviolet at the skin portion to be investigated, to collect the reflected radiation and to analyse the autofluorescence spectra of the skin by means of a spectrophotometer. A microcontroller allows to examine all spectral components, without limiting to specific wavelength ranges, by means of the phasor method. The application of the phasor method returns, as result for each analysed spectrum, the values of the cosine and sine components, which are represented as a point on the phasor plane. The mapping of the phasor plane allows to associate immediately the position of the point in the plane under pathological dermatological conditions.

FIG. 1

EP 4 755 293 A1

## Description

## Technical field of the invention

The present invention relates to a portable system for diagnosing the health status of a patient's skin according to a not invasive mode, based on the analysis of the skin autofluorescence spectrum.

The system is configured to emit an excitation light radiation in the ultraviolet at the skin portion to be investigated, to collect the reflected radiation and to analyse the skin autofluorescence spectra by exploiting the phasor method, by providing accurate and quantitative data about the skin pathophysiological conditions.

## Background

The skin is the most extended organ of the human body and performs an important protective role against the physical, chemical and biological agents. Since it is an external organ accessible in a minimally invasive way, in the history of medicine the skin has been the subject of several optical investigation studies with the purpose of monitoring the integrity and the health status thereof.

The light radiation interacts with specific molecules present in the skin, known as chromophores. These, thanks to their particular molecular structure, are capable of absorbing selectively determined wavelengths, by converting the radiation energy into chemical energy or heat.

In particular, some chromophores are also fluorescent, that is after absorbing radiative energy at a determined wavelength, they are capable of re-emitting part thereof at higher wavelengths, but with an emission intensity 1000 times lower than the intensity of the incident radiation.

The skin chromophores are biomolecules of pathophysiological interest, thereamong it is possible to mention collagen, elastin, nicotinamide adenine dinucleotide (NAD or NADH, depending on the oxidation status), urocanic acid and the Advanced Glycation End-product (AGE). AGE is a group of heterogeneous compounds produced by the not enzymatic attachment of glucose to proteins, lipids or nucleic acids, through Maillard reaction, which alters significantly their function. In other words, AGEs are advanced glycation end products, which accumulate in tissues and are associated to the aging process and various chronic diseases such as diabetes. It has been widely demonstrated that the accumulation of AGE determines adverse structural and functional modifications of the tissues. For this reason, up to now several studies have concentrated on the quantification and evaluation of AGEs to predict metabolic diseases or complications linked thereto.

In this sense, it is known that several devices for measuring the specific skin autofluorescence of AGEs (*AGE reader*) have been devised. Autofluorescence is the propriety of some molecules to emit light (fluorescence) when excited by a specific wavelength, as already represented.

One of such devices of known type is substantially based on a measurement unit which uses a UV fluorescence lamp, enclosed in a shielding structure, which emits UV light on the skin to be investigated. An optical fibre collects the autofluorescence emitted in response by the skin and transmits it to a spectrophotometer, through which it is possible to measure a ratio between the autofluorescence values detected in two emission ranges. This ratio is then correlated to the AGE content in the area of irradiated skin.

Such device was tested in a clinical context, on two groups of participants: one consisting of diabetic patients and another one of healthy individuals, used as control group. The results showed that the diabetic patients had significantly higher autofluorescence levels than the healthy control group. An increase correlated to age in the autofluorescence levels in diabetic patients was observed, by suggesting a progressive accumulation of AGE over time. Moreover, a strong correlation between the autofluorescence levels and those of HbA1c was detected, indicating a less effective glycaemic control.

However, the above-mentioned device of known type calculates the autofluorescence index as a ratio between two emission intervals, by taking into consideration one limited part of the spectrum only. This approach limits the capability of evaluating other potential markers or factors which could influence the characterization of the dermal phenotype. It is noted that each biomolecule contributes in a unique way to the structure, to the function and to the response of the skin to external stimuli. Therefore, the importance of all species present at skin level and their related composition is essential, in order to evaluate completely and accurately the health of the patient's skin.

In more general terms, the technologies nowadays available for the not invasive evaluation of AGE accumulation in the skin, with a particular relevance for the metabolic pathologies such as diabetes, have some important limits, shown hereinafter.

Limitation of the measurement area: the already known solutions require that the patient's forearm is positioned inside the device to perform the measurement. This configuration limits the body area therefrom the measurements can be obtained, by reducing the possibility of a complete and accurate evaluation of AGE levels in different body parts. Since AGE concentration varies in the different body regions and depending on and in presence of even local pathologies, the fact of measuring some skin areas only (for example the forearm) might not provide an accurate representation of the general pathophysiology of the patient.

Limited spectrum: the known solutions calculate the autofluorescence index like a ratio between the autofluorescence radiation in two emission ranges, by taking into consideration one limited part of the spectrum only. The evaluation of other potential markers or factors which could influence the AGE levels is not provided. The fact of concentrating on a restricted part of the spectrum reduces the capability of detecting other components, which on the contrary would be necessary for a complete and accurate evaluation of the patient's skin.

**Summary of the invention**

The technical problem placed and solved by the present invention is then to provide a solution allowing to obviate the drawbacks mentioned above with reference to the known art.

The invention relates to a system configured to emit an excitation radiation, to detect an autofluorescence radiation emitted in response by the skin and to analyse its spectrum by means of the phasor method to detect skin pathologies, as defined in the independent claim 1.

Preferred features of the present invention are set forth in the depending claims.

The proposed system not only allows a quick and not invasive measurement of the skin autofluorescence, but also allows to evaluate, through the study of the emission spectrum, the contribution of all species of biomolecules present in the investigated skin, with the purpose of determining the health status of the skin.

The proposed system can be of portable type and it substantially comprises an ultraviolet excitation LED light source, an optical fibre bearing a bifurcated terminal end to allow the transmission of the excitation to the skin and the collection of the light emitted in response by the skin, a high-resolution spectrophotometer and a microcontroller to perform the analysis of the acquired data. The analytical technique which measures how much a substance absorbs light at different wavelengths is called spectrophotometry. The spectrophotometer is an instrument used to measure the light intensity as a function of the wavelength, to analyse the absorption or emission of light by a sample.

In the system of the invention, the optical fibre, after having emitted the excitation radiation, collects the autofluorescence emitted by the investigated skin and transmits it to the spectrophotometer, through which it is possible to measure a ratio between the autofluorescence values detected in two emission ranges. The above-mentioned ratio is correlated to the content of AGE and/or other species of biomolecules in the area of irradiated skin.

The microcontroller is configured and programmed to examine all spectral components, without limiting to specific ranges of wavelengths. The application of the phasor method returns as result, for each analysed spectrum, the values of cosine and sine (respectively designated as G and S), which are used to quantify the differences between the detected spectra under investigation conditions and those already known related to reference conditions of healthy patients. Such components G and S are represented as a point on the phasor plane, which can be shown on video to the user. The mapping of the phasor plane allows to associate immediately the position of the point in the plane under inflammatory dermatological conditions. Such analysis of the spectral data further allows to apply clustering techniques to characterize different conditions linked to the health status of the skin.

According to an advantageous aspect, the proposed system further includes an ergonomic handpiece for guiding and supporting the optical fibre, which can be made of hypoallergenic material. The handpiece allows to position precisely the fibre on the skin area of interest. The handpiece shape can allow to vary the orientation of the optical fibre even without moving the handpiece itself, thanks to the presence of flexible connecting elements which connect rigid bodies, bearing seats for housing the optical fibre. Moreover, the handpiece is physically separated from the spectrophotometer and from the microcontroller, thereto it is connected exclusively by means of the optical fibre. Such configuration allows to use the handpiece with extreme freedom of movement and to investigate any portion of the patient skin.

Additional advantageous features of preferred embodiments of the proposed system are inherent in its flexibility and portability, by allowing accurate measurements on any body district, for a complete diagnosis of the dermatological and metabolical pathologies.

In fact, differently from the already known solutions, which consider only one limited part of the spectrum, the proposed system uses a spectrophotometer which allows to quantify the intensity of the different collected wavelengths. This improves the capability of detecting a wider range of biomarkers. The system further allows to control the output of the excitation light emitted by the LED to optimize the signal-noise ratio based on the specific clinical needs.

The system for analysing data used by the system is based upon the phasor theory, a mathematical instrument to decompose the periodic signals into sinusoidal and cosinusoidal components. In the proposed system, the phasor theory is used to analyse the collected fluorescence signals. The integration of this approach in an analytical hardware allows to explore deeply and in real time the whole emission spectrum of the skin, without limiting the analysis to ranges of wavelength and, thus, to specific biomolecules. This allows to highlight the structure and the relations present in the collected data, by providing significant information for prevention, diagnosis and therapy.

More in particular, the phasors are applied as instrument for clustering spectra related to different dermatological pathologies. Such use of the phasor technique for identifying and classifying dermatological conditions through the autofluorescence spectrum introduces a substantial progress with respect to the solutions known to the state of art. Moreover, it is confirmed that the proposed invention does not limit to calculate a ratio between two spectral ranges to measure the AGE accumulation, but it examines the whole autofluorescence spectrum. This allows to evaluate not only AGEs, but also several other autofluorescent species present in the skin (such as collagen, elastin, NADH, etc.), thus by improving accuracy and depth of the diagnosis. In other words, the integration of the phasor theory with the analysis of a wide spectrum of autofluorescent species provides an advanced diagnostic system, capable of distinguishing with precision between different dermatological pathologies.

More specifically, the microcontroller can be programmed to process the skin autofluorescence spectrum by means of an algorithm which exploits machine learning techniques, by allowing an in-depth analysis of the whole emission spectrum of the skin, by highlighting the structure and the relations present within the collected data. Such implementing mode represents a significant progress in monitoring the pathophysiological status of the skin, by offering an overall vision of the dermatological conditions, that goes beyond the more simplistic approaches based on one single parameter.

The variants having an advanced integration of the analytical hardware in an integrated circuit in the system provide feedback on the skin health status in real time.

Moreover, the system can have a compact configuration which makes it particularly easy to be transported, by allowing the use thereof in different clinical, and not clinical, contexts, without the need for a fixed location.

The system then is configured to offer significant advantages for a wide range of users (healthcare professionals, researchers, consumers), by improving the diagnosis and monitoring of the skin conditions. This is possible through flexible, accurate and not invasive measurements.

The system in particular allows to evaluate the action and the effectiveness of cosmetic products, as well as the follow-up of dermatological and metabolic pathologies, thus representing a significant progress with respect to the traditional methods.

One among the most interesting aspects is the system flexibility and portability, with particular reference to the handpiece, which allows accurate measurements on any part of the body. This feature makes the proposed system interesting for a wide spectrum of users, from the professionals of the healthcare field to the researchers, up to the consumers interested in monitoring the skin health or the follow-up of dermatological and metabolic pathologies.

Finally, the system provides as output quantitative and detailed analyses of the skin conditions, with the possibility of identifying pathological markers early and evaluating the effectiveness of the cosmetic products.

Other advantages, features and use modes of the present invention will result evident from the following detailed description of some embodiments, shown by way of example and not for limiting purposes.

**Brief description of figures**

The figures of the enclosed drawings will be referred to, wherein:

- Figure 1 shows a schematic view of a first preferred embodiment of a system according to the invention;

- Figures 2 and 3 show a schematic view in longitudinal section of a preferred embodiment of a handpiece included in the system of Figure 1, wherein the flow of the excitation radiation and the one of the autofluorescence radiation, respectively, is represented;

- Figure 4 shows a perspective view of a preferred embodiment of a handpiece included in the system of Figure 1;

- Figure 5 shows a front view of a preferred embodiment of a handpiece included in the system of Figure 1, in exploded configuration;

- Figure 6 shows in detail a perspective view of the free terminal end of the handpiece of Figure 5, in exploded configuration;

- Figure 7 shows a schematic front view of a preferred embodiment of a microcontroller with integrated display included in the system of Figure 1;

- Figure 8 shows a schematic representation of the evaluation of the anti-glycant efficacy of a dermatological cream performed by means of the system according to the present invention;

- Figures 9 and 10 show phasor analysis schemes of autofluorescence radiations emitted by the skin of a patient thereto a cream with active principle and a control cream, respectively, is applied;

- Figures 11 and 12 show a representation of spectral profiles of autofluorescence radiations emitted by the skin of a patient thereto the cream with active principle and the control cream, respectively, is applied;

- Figure 13 shows a graphical representation of the phasor analysis of autofluorescence radiations emitted by the skin of a patient thereto the cream with active principle and the control cream is applied;

- Figure 14 is an enlargement of the central portion of Figure 13;

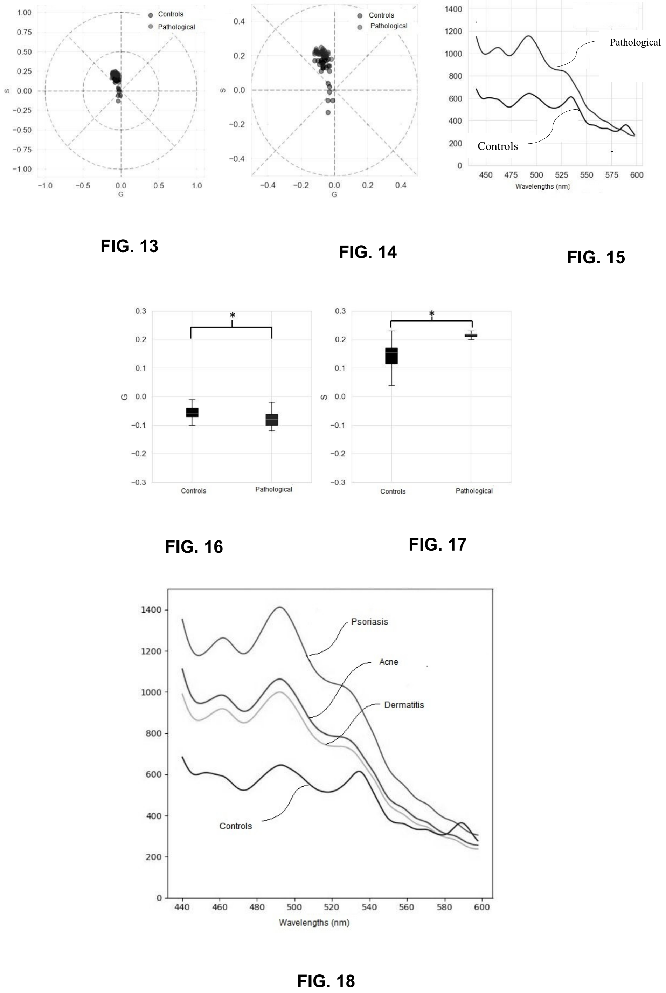

■ Figure 15 shows a representation which compares the spectral profiles of autofluorescence radiations emitted by the skin of a patient thereto the cream with active principle is applied and by the skin of a patient thereto the control cream is applied;

■ Figures 16 and 17, respectively, show phasor analysis schemes of autofluorescence radiations emitted by the skin of a patient thereto the cream with active principle is applied and by the skin of a patient thereto the control cream is applied, in comparison;

■ Figure 18 shows a representation of the spectral profiles of skin autofluorescence associated to different pathologies;

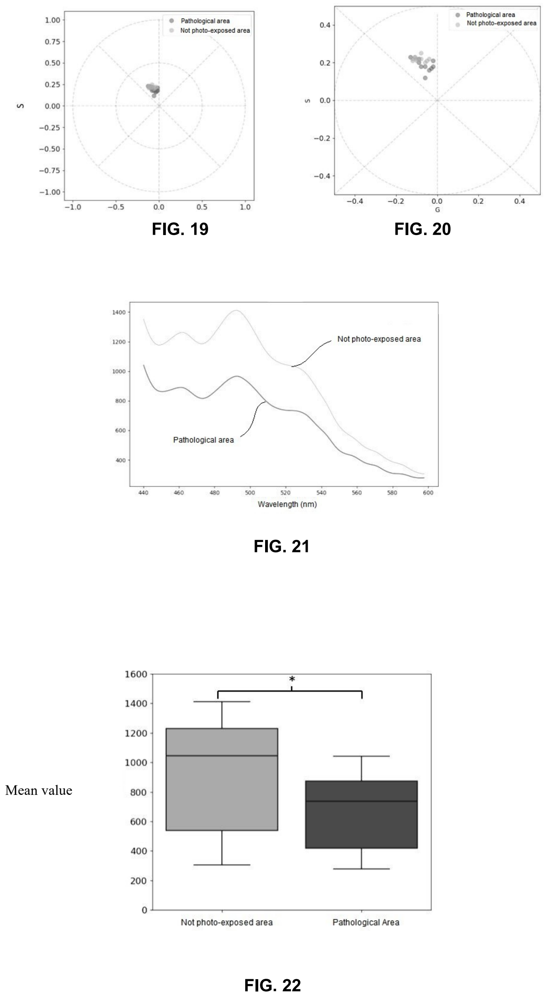

■ Figure 19 shows a graphical representation of the phasor analysis of autofluorescence radiations emitted by a pathological area of a patient's skin and by a not photo-exposed area of a patient's skin, in comparison;

■ Figure 20 is an enlargement of the central portion of Figure 19;

■ Figure 21 shows a representation of the spectral profiles of skin autofluorescence associated to the pathological area of a patient's skin and to the not photo-exposed area of a patient's skin;

■ Figure 22 shows phasor analysis schemes of skin autofluorescence radiations associated to the pathological area of a patient's skin and to the not photo-exposed area of a patient's skin.

The sizes, thicknesses and curves represented in the above-illustrated Figures are to be meant as exemplifying, they can be magnified and they are not necessarily shown in proportion.

**Detailed description of preferred embodiments**

The skin autofluorescence varies significantly between healthy individuals and those affected by inflammatory pathologies of the skin, due to the presence and the concentrations of specific fluorophores. The main fluorophores responsible for the skin autofluorescence include collagen, elastin, NADH (nicotinamide adenine dinucleotide) and Advanced Glycation End Products (AGE). These molecules have fluorescent properties which change in response to the physiological and pathological conditions of the skin.

In particular, collagen and elastin, main components of the connective tissue of the skin, may result to be degraded and disorganized under pathological conditions, by determining a variation in their autofluorescence spectra. The disorganization of collagen, for example, is a marker of chronic inflammation and skin aging. NADH is a coenzyme involved in the cellular metabolic processes. The variation in the levels of NADH can show metabolic alterations in the skin cells, often observed in inflammatory states and skin diseases such as psoriasis and acne. AGEs are stable products formed through not enzymatic reactions between reducing sugars and proteins or lipids. They accumulate in the cells and tissues over time and they are known to contribute to various pathological conditions, including the skin disorders. AGEs have a strong autofluorescence and their accumulation in the skin is correlated to inflammatory processes and chronic diseases such as diabetes and atherosclerosis. In the skin, AGEs can link to the specific receptors (RAGE), by triggering a cascade of inflammatory events which alter the skin structure and function. The skin chronic inflammation, like the one observed under conditions such as psoriasis, acne and atopic dermatitis leads to changes in the composition and organization of the skin fluorophores. The inflammatory cells, the inflammatory mediators and the oxidative stress contribute in modifying the biochemical environment of the skin, thus influencing the autofluorescence profiles.

The system of the invention, by analysing the whole autofluorescence spectra, is capable of detecting the differences between the autofluorescence profiles and the profiles associated to physiological situations in which there is absence of pathologies (already known by means of the detection of experimental data used for setting the system), which reflect the biochemical and/or structural alterations induced by skin pathologies, by allowing the use of autofluorescence as biomarker for diagnosing and monitoring the dermatological conditions.

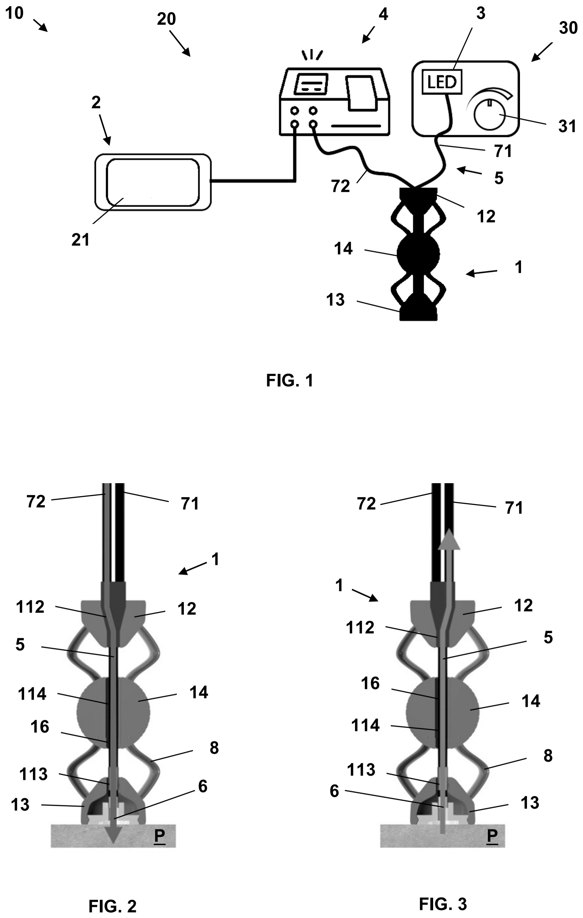

By firstly referring to Figure 1, a preferred embodiment of a system for diagnosing the health status of a patient's skin according to the theories and the action mechanisms illustrated above is designated as a whole with 10.

The proposed system allows to obtain feedback, even in real time, with respect to the presence of pathologies affecting the portion of investigated skin (or even more generally, of pathologies afflicting the patient), according to an absolutely not invasive mode which exploits the principle of the skin autofluorescence.

To this purpose, the system 10 comprises an excitation unit 30, configured to generate a radiation with which the portion of target skin P is to be hit, and a processing unit 20, configured for analysing the autofluorescence spectrum emitted by the irradiated skin to detect possible pathologies. The above-mentioned portion of skin P can be meant as a reference plane with respect thereto the excitation radiation is preferably arranged orthogonally, in use.

The excitation unit 30 comprises a light source 3 configured to generate and emit the excitation light radiation, which is preferably an ultraviolet radiation (UV). The light source 3 can include a light emitting diode, for example LED (*Light Emission Diode*). The excitation wavelength can be adjustable, for example variable in a range from 280 nm to 1450 nm.

The excitation unit 30 can comprise a control device 31 for adjusting the output of the excitation light emitted by the light source 3. The preferred configuration allowing to vary the excitation wavelength thanks to the system which uses LED light, allows to explore the autofluorescence emitted by different biomolecules with different excitation wavelength. According to such variant, it is possible to widen the system analytical capabilities, by improving the precision in the diagnosis and characterization of the skin conditions. The system can further include filtering means configured so that only the light with wavelengths between 300 and 420 nm reaches the skin P.

By way of example, the technical specifications of a LED light source suitable to be used in the proposed system are shown hereinafter:

Colour: UV

Nominal wavelength: 385 nm (with possibility of varying the excitation wavelength from 280 nm to 1450 nm)

Bandwidth (FWHM): 12 nm

Maximum current (CW): 1400 mA

Electrical power: 5110 mW

Emitter size: 1.4 mm x 1.4 mm

Direct voltage: 3.65 V

The power of the LED diode can be adjusted through a dedicated driver connected to the light source, which allows to manage the intensity of the outgoing light radiations.

The system 10 further comprises, as anticipated, a processing unit 20, which includes a spectrophotometer 4 and a microcontroller 2.

The spectrophotometer 4 is configured to implement a spectrophotometric analysis, that is to determine the electromagnetic spectrum of the autofluorescence light radiation, which constitutes an incident radiation emitted by the portion of skin P irradiated by means of the excitation unit 30. The investigated emission range can go from the ultraviolet (400 nm) to the infrared (4000 nm). The spectrophotometer 4 is an optical system configured to collect, discern and quantify the intensity of the different wavelengths of the autofluorescence light radiation. It may consist of a diffraction grating which allows to separate spatially the different wavelengths. These light components then can be detected by a linear array of diodes downstream of the grating.

By way of example, a spectrophotometer suitable to be used in a system according to the invention can be provided with 14 different diffraction gratings, from the UV to the near infrared, which vary based upon the density of lines per millimetre. The detector used in such spectrophotometer is a high-sensitivity linear CCD array with 2.048 elements, the specifications thereof are shown hereinafter:

Number of elements: 2.048 pixels

Pixel size: 14 $\mu$m x 200 $\mu$m

Pixel well depth: 62.500 electrons

Signal/noise ratio: 250:1 (with full signal)

Resolution A/D:12 bit

Dark noise: 2.5 RMS counts

The microcontroller 2 is configured to analyse the spectrophotometric data obtained by means of the spectrophotometer 4. Therefore, a data connection is provided between the spectrophotometer 4 and the microcontroller 2.

The microcontroller 2 can be in communication with the excitation unit 30, in particular to adjust and command the activation of the light source 3 and/or of the control device 31.

The microcontroller is configured to process at hardware level the autofluorescence spectra through the phasor method, which allows to extract the cosine and sine components (coordinates G and S) of each spectrum, by representing them as a point in a two-dimensional plane (phasor plane or Gauss plane). In other words, the microcontroller 2 is configured to process all spectral components of the electromagnetic spectrum by means of the phasor method, by decomposing them in cosinusoidal components G and sinusoidal components S and by representing them on the phasor plane.

The position of this point in the phasor plane reflects the spectral features of the autofluorescence spectrum of the skin, by allowing the system to evaluate quickly the presence or the predisposition to specific dermatological and metabolic conditions.

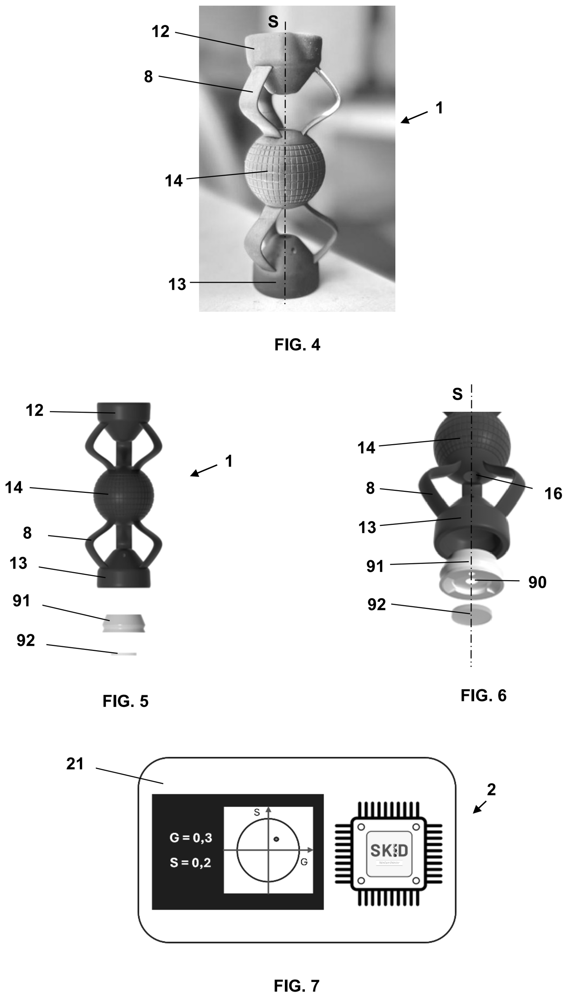

This approach provides simple metrics and allows a quick quantification of data and the clustering of pathophysiological states. The system 10 can further allow to display the autofluorescence radiation spectra directly on a display 21 included in the processing unit 20, which can be integrated in the microcontroller 2 as shown more in detail in Figure 7, or on an external screen connected to the microcontroller for example through HDMI.

In summary, the microcontroller 2 is configured to receive as input the spectrophotometric data from the spectrophotometer 4, to process at hardware level the autofluorescence spectra through the phasor method and to provide as output the coordinates G and S of each spectrum (cosine and sine components), by representing them as a point in a two-dimensional plane. The value of the coordinates and the corresponding point in the plane can be shown on a display, as anticipated.

The microcontroller 2 is further configured so as to map the Gauss plane and to associated the position of the cosinusoidal (G) and sinusoidal (S) components represented therein with a biomarker and/or an inflammatory dermatological condition, according to association criteria (algorithms) stored in the same microcontroller 2. Such criteria in particular include that:

> one or more wavelengths comprised in the autofluorescence spectrum are associated to the presence of a respective biomarker, and

> each possible position of the cosinusoidal (G) and sinusoidal (S) components of such specific wavelengths associated to a respective biomarker are associated to the presence or absence of a pathology.

The microcontroller 2 in particular is configured to quantify the intensity of a plurality of wavelengths included in the autofluorescence spectrum, and then a plurality of biomarkers, thereamong AGE, collagen, elastin and/or NADH.

Moreover, the microcontroller 2 can be configured to store the data on the components of the autofluorescence electromagnetic spectrum, as well as the processing results, including the possible detected dermatological pathologies.

As far as the conveying of the excitation light radiation and autofluorescence is concerned, an optical fibre 5 is used.

To this purpose, the optical fibre 5 comprises a free distal terminal end 6 and a bifurcated proximal terminal end 7. The distal end 6 is implemented as a single and unitary body, whereas the opposite terminal end is divided into two parts, and it includes a first end 71, arranged at the light source 3, and a second end 72, connected to the processing unit 20, in particular to the spectrophotometer 4. Therefore, for transmitting and collecting the light radiation one single optical fibre is used. The fibre is equipped with a dedicated optical system for inserting the light into the fibre (*launcher*) and for its collection (*emitter*), by guaranteeing the generation of a uniform wave on the skin surface. The fibre allows to obtain a radiation beam with reduced sizes (ex. about one millimetre) by guaranteeing a detailed analysis of extremely reduced areas.

The section of not bifurcated fibre is inserted in the handpiece and it sends light radiation on the skin surface. The two bifurcated ends of the fibre connect to the diode and to the spectrophotometer, respectively (Figure 1).

The optical fibre 5 is configured: to convey the excitation light radiation produced by the light source 3 by means of the first end 71, and to emit it by means of the distal terminal end 6 at the portion of the patient's skin P, as well as further to collect the autofluorescence light radiation emitted by the portion of skin P - irradiated by means of said distal terminal end 6 - and to convey it towards the processing unit 20 by means of the second end 72. In Figures 2 and 3 the two radiation paths, respectively sent by the emitter (Figure 2) and emitted by the skin (Figure 3), are highlighted.

In order to support and guide the optical fibre 5, the system 10 comprises a handpiece 1, configured to be able to be grasped and maneuvered by an operator. The excitation unit 30, the processing unit 20 and the handpiece 1 can be physically separated and not integral with each other (unless the optical fibre and/or other type of cables), that is with mutual freedom of movement and/or not included, or integrated, in a common fixed frame.

The handpiece 1 comprises a pass-through seat 16 configured to house the optical fibre 5. Preferably, the handpiece 1 has an elongated shape, aimed at receiving the optical fibre for its whole length, in longitudinal direction, and to this purpose it has an input opening and an output opening respectively obtained at a first terminal end of the handpiece 1 and a second terminal end of the handpiece 1.

More in detail, the handpiece 1 can include a plurality of bodies arranged in series and having a through-opening, each body being connected to the immediately preceding body and to the immediately subsequent one through connecting elements, which preferably are deformable, for example flexible and/or elastic, to allow the mutual misalignment between the bodies. Such aspect provides flexibility and agility in using the system 10, since it allows to vary the orientation of the optical fibre 15 without altering the guiding and supporting functions offered by the handpiece 1. One or more bodies comprised in the handpiece 1 preferably have a spherical or ovoidal shape, which makes immediate and ergonomic the manipulation thereof, and they can consist or be coated with a hypoallergenic material. Such design of the handpiece 1 is suitable to ease the sterilization thereof and to guarantee an optimum positioning of the optical fibre 5 on the patient's skin, by guaranteeing hygiene and optimum acquisition of the signal, to allow high quality of measurements.

According to a preferred variant shown in Figures 2 to 4, the handpiece 1 comprises a proximal body 12, a distal body 13 and a central body 14 interposed between such proximal body 12 and distal body 13, where under distal the end intended to be arranged near the portion of the patient's skin to be investigated P, in use, is meant. The central body 14 can have a spherical shape.

Each one of the proximal, distal and central bodies 12, 13, 14 has a respective passage span 112, 113, 114 configured to house the optical fibre 5. The proximal, distal and central bodies 12, 13, 14 are configured to be arranged in series, such that the respective passage spans 112, 113, 114 are aligned to each other. As shown in the enclosed Figures 2-6, the handpiece 1 comprises flexible connecting elements 8 arranged to connect the central body 14 with the proximal body 12 and the distal body 13, respectively, by allowing the mutual movement thereof (within limits associated to the capability of the connection elements to deform).

Preferably, the pass-through seat 16 is arranged centrally with respect to each one of the proximal, distal and central bodies 12, 13, 14 and it can include a central axis of symmetry of the above-mentioned bodies, by way of example shown and designated with S in Figures 4 and 6, which can coincide with an axis of symmetry of the seat 16 itself.

According to additional advantageous aspects, at the distal terminal end of the handpiece 1, that is of the free end intended to face the portion of the patient's skin to be investigated, in use, it is possible to provide a removable optical window configured to reduce the angular dispersion of the emitted excitation light radiation, by improving the transportation and collection of the fluorescence radiation and by reducing signal losses.

Such optical window can be implemented by installing at the free end of the handpiece 1 two supplementary components, shown by way of example in Figures 5 and 6. The first component, which can be arranged inside the handpiece 1, for example assembled in a respective seat, is preferably made of thermoplastic polyurethane (TPU). More generally, the first component is made of a flexible material, resistant to impacts, to UV rays, to water and to chemical agents. The first component has an opening for the passage of the optical fibre 5, configured with the purpose of improving the positioning thereof inside the handpiece 1. The second component, instead, can be a thin disk which can be made of plexiglass, with the function of easing the transportation and collection of fluorescence radiation without absorbing the UV radiations and in the visible spectrum.

With reference to the embodiment shown in Figures 5 and 6, the distal body 13 can include a free terminal end intended to face the portion of the patient's skin P, thereat the above-mentioned first component, such as a centring element 91, having a through-hole 90 for positioning said optical fibre 5, and the above-mentioned second component, such as a protection element 92, suitable for closing the through-hole 90, are arranged. The protection element 92 is transparent to the excitation light radiation and to the autofluorescence light radiation. Such centring and protection elements 91, 92 are arranged in series, in particular along the axis S, and the protection element 92 is arranged more externally with respect to the centring element 91.

The present invention further provides a method to determine the pathological or physiological condition of a portion of a patient's skin in a not invasive way, according to the already illustrated peculiarities.

The method first of all provides to irradiate the skin portion by means of an excitation light radiation, then to collect an autofluorescence light radiation emitted 'in response' by the same skin portion. Secondly, it is provided to determine the electromagnetic spectrum of the above-mentioned autofluorescence light radiation.

An additional phase is to process all spectral components of the electromagnetic spectrum as determined by the phasor method, by decomposing them in cosinusoidal (G) and sinusoidal (S) components and by representing them on the Gauss plane.

A mapping of the Gauss plane is then performed and the positions of the cosinusoidal (G) and sinusoidal (S) components represented therein are associated to concentration and/or distribution values of a respective biomarker, as a function of already known/predetermined association data. Depending on the type of biomarker and its concentration and/or distribution, a physiological condition or a pathological condition of the patient's skin is determined.

The use of the phasor technique for the diagnostic clustering of different dermatological pathologies, in combination with the analysis of the whole autofluorescence spectrum, allows to evaluate several skin biological markers apart from AGEs, generally unique target of the examinations of autofluorescence of known type.

With reference to a preferred variant, the invention provides a method for diagnosing the skin health status based on the autofluorescence analysis, which can be articulated in the following steps:

- Step 1: Data Acquisition

    ◦ In order to acquire spectra, the handpiece is positioned on the skin surface of interest, by making sure that the area is clean and without impurities or cosmetics. The radiation is emitted by the LED and transmitted through the optical fibre (Figure 2).

- Step 2: Processing and Analysis of Data in real time

    ◦ The autofluorescence emitted by the skin is collected by the optical fibre and transmitted to the Spectrophotometer (Figure 3).
    ◦ The whole collected spectrum is analysed by using the phasor method by the dedicated microcontroller.

- Step 3: Output and Feedback

    ◦ The analysis method returns the values of the components G and S for each spectrum and such values are shown to the user on the integrated screen or sent to an external screen.
    ◦ Through a mapping of the phasor plane, which allows to lead a determined spectrum back to a specific condition, immediate feedback is provided to the user.

The potential of the system and of the method proposed by means of the present invention were tested, at laboratory level, in two main fields: the cosmetological one and the dermatological one, according to the herebelow illustrated experiments.

*Cosmetic Field: Anti-glycation Efficacy Test*

In cosmetological field, the system according to the invention was used in our laboratory to perform an efficacy test on a cosmetic product specially formulated to contrast the glycation process, a phenomenon that is increasingly known and studied for its ability to cause tissue damages, contributing to aging.

In cosmetics, the anti-glycation activity can be evaluated in vitro, through tests based on the capability of a substance to prevent the formation of glycated proteins or inhibiting the formation of AGE.

In order to perform anti-glycation efficacy test 6 healthy volunteers were recruited, exclusively women, with an average age of $27 \pm 3.4$ years, including 2 smokers (ID1 and ID2) and 4 non-smokers.

In the preliminary step, the two creams to be compared were prepared. The INCIs of the products used in the test are the following:

- "Anti-Glycation Serum Elixir of Youth" ingredients: Water (Aqua), Glycerin, Sorbitol, Phospholipids, Coconut Oil Polyglyceryl-6 Esters, Xanthan Gum, Aspergillus Ferment, Tocopheryl Acetate;

- "Moisturizing Base Cream" ingredients: water, butyrospermum parkii butter, glycerin, hydrogenated ethylhesyl olivate (e) hydrogenated olive oil unsaponifiables, helianthus annuus seed oil, argania spinosa kernel oil, thylhexyl stearate, glyceryl stearate, ceteraryl alcohol, stearic acid, sodium lauroyl glutamate, dicaprylyl ether, cethyl alcohol, tocopherol, caesalpinia spinosa gum, sodium benzoate, potassium sorbate, sodium dehydroacetate, benzyl alcohol, lactice acid.

Firstly, 250 ml of "Moisturizing Base Cream" were put in a becher and mixed for few minutes with a spatula. By using a graduated pipette, 2.5 ml of " Anti-Glycation Serum Elixir of Youth " were added, in order to keep the concentration in the range of 0.5-1%. 6 50-ml cosmetic bottles were filled up after having mixed again for few minutes and having guaranteed the preparation homogeneity. The glass containers, provided with dispensing small pump, were at last labelled as "Cream A", to show the potential activity. The same filling procedure was performed, thanks to a graduated syringe, for the other 6 bottles containing the base cream only and then labelled as "Cream C", showing the control.

The study required the participants to stop for 2 weeks (14 days) any cosmetic treatment different from their usual cleaning and to apply only the two creams provided on the first day of measurements (T0). At least 2 weeks before starting the study, the participants were requested to suspend the use of suncreams and self-tanning products. The make-up was not subjected to particular restrictions, except in case of products that promised anti-aging action.

The creams were then applied for 14 days, twice a day (morning and evening), on face and neck, after cleaning with the usual detergent. The comparison was possible by applying Cream A on the right portion of face and neck, whereas the control was applied specularly on the left area.

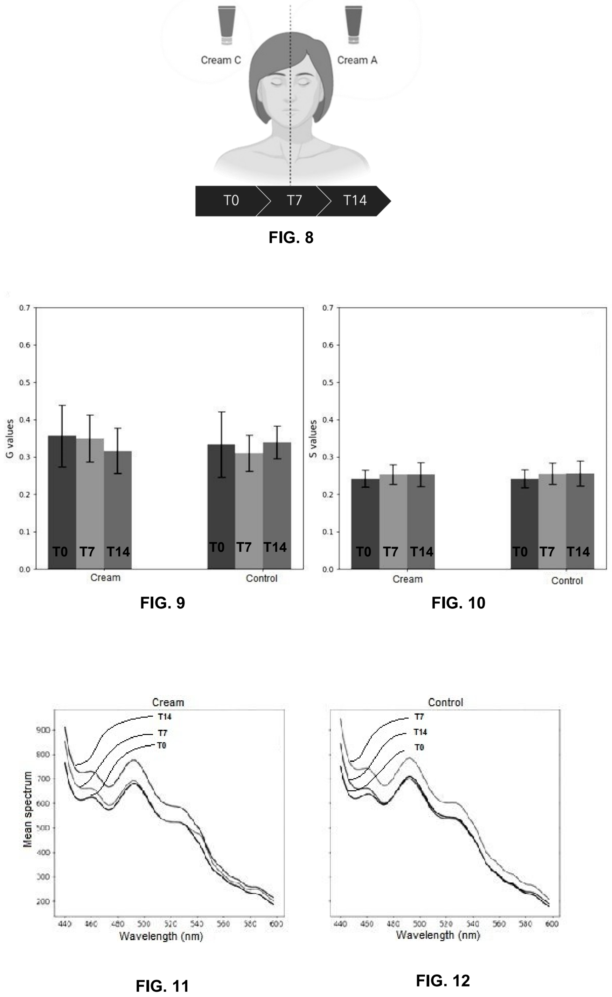

The measurements of the the skin autofluorescence were performed with the invention system at T0, before starting the treatment, after one application week (T7) and at last on day 14 (T14). At each measurement the volunteers were requested to show up after having followed the skin check-up preparation method: cleansing face and neck on the preceding evening (or at least 6-8h before measurement) and avoiding the application of any cosmetic. An experiment scheme is represented in Figure 8, which provides a schematic representation of the evaluation of the anti-glycant efficacy of a dermatological cream.

In brief, each participant was asked to apply two different creams on the two sides of their face, respectively the control cream (C) on the right portion, and the cream containing the active principle (A) on the left portion of the face, for 14 consecutive days. The autofluorescence spectra were collected with the invention system before starting the application (T0), after 7 days (T7) and after 14 days of daily application (T14). The acquisition parameters, during measurements, were the following ones:

  ○ Laser power: 0.2 A;

  ○ Integration time: 1000 ms.

The enclosed Figures 9 to 12 show the results obtained by applying the phasor algorithm on the spectra collected during experiments. Specifically, the forehead measurement area where on the right part the control cream was applied (Control in the graphs) was considered, whereas on the left part a cream with active principle (Cream in the graphs) was applied at three measurement instants (timepoints) or T0, T7 and T14. Specifically, such Figures show the results of the phasor analysis and the spectral profiles of the area being tested, in particular the forehead area, by applying active cream (Cream) and control cream (Control) at the three timepoints T0, T7 and T14, considering the data of the six people involved in the study.

In Figures 9 and 10, respectively the mean values of the coordinates G (Figure 9) and S (Figure 10) for the area with the application of the cream with active principle (Cream) and area with control cream (Control) in the three measurement timepoints, respectively T0, T7 and T14, are shown. The results show a decrease in the values of the coordinate G for the area treated with the active cream with respect to the area treated with the control cream. On the contrary, no significant variations in the values of the coordinate S were observed. This could suggest an effect of the treatment with the active principle on the variation in the spectrum of the treated area.

Figures 11 and 12 show respectively the mean spectra in the range of wavelengths 440-600 nm for the area where the cream with active principle (Cream) and the control cream (Control) in the three measurements timepoints (T0, T7, T14) were applied. In Figure 11, an increase in intensity of the spectra associated to the active cream is evident as the days of treatment increase, by suggesting a cumulative effect of the treatment on the spectral profile and a more significant biological response. On the contrary, in case of the control cream (Figure 12), no significant changes in the intensity of spectra over time are observed, showing that the control cream has no substantial effect on the spectral profile in the considered period. By considering this spectral range it is possible to calculate the AGE index (also called Skin AutoFluorescence, SAF), expressed in arbitrary units (AU) as ratio between the intensity of the emission light in the range 450-600 nm and the intensity of the excitation light between 420-440 nm which quantifies the spectral changes. The values of the SAF index obtained under these conditions show a significant decrease in the area treated with the active cream with respect to the control area. This confirms the treatment efficacy and demonstrates that the cream application has a measurable and positive impact on the skin over time.

*Dermatological Field: Clinical Screening on skin inflammatory pathologies*

In dermatological field, the invention system was tested in the context of a clinical screening. The adhesion to the study occurred on a voluntary basis. In the initial step, specific questions were asked to the subjects with the purpose of acquiring anagraphical data and information about their health (healthy or pathological) state, their lifestyle (smoker, active or sedentary lifestyle, etc.) and the habits in treating their skin (ex. use of creams or cleansers).

For this screening 26 volunteers were selected, previously subjected to clinical diagnosis by professional dermatologists, who had a variety of skin conditions.

For the analysis the autofluorescence on the internal segment of the arm was evaluated.

For the acquisitions, a system according to the present invention was configured as follows:

◦Laser power: 0.2 A

◦Integration time: 1000 ms

Through the application of the phasor method, the control and the pathological subjects in the phasor plane in the measurement area, that is not photo-exposed area (arm), shown in Figures 13 to 17, were represented. The considered pathological clinical conditions were acne, atopic dermatitis and psoriasis. By observing the phasor plane in case of the not photo-exposed area (Figure 13 and respective enlargement of Figure 14), it is possible to note that the spectra of the pathological subjects concentrate in an area above controls. It is then possible to distinguish visually the two (healthy and pathological) groups which cluster distinctly in the phasor plots, by highlighting a significant difference in the skin autofluorescence.

In order to quantify the statistical significance of the differences, box plots for the values of G and S of the two groups are reported, which have significant differences ($p<0.01$). Even by observing the mean spectra (Figure 14), it is noted that the two groups have clearly distinguishable spectral profiles in the considered range of wavelengths (420-600 nm). Even from the boxplots of Figures 15 and 16 it is possible to observe significant differences ($p<0.005$) for the coordinates G, S of the two groups, after applying t-test and Mann-Whitney U test.

In the representation of the mean spectra of the two areas shown in Figure 18, the pathological subjects have higher intensity values with respect to controls. The first ones could have biological alterations which lead to an increase in intensity of the signals, by suggesting an anomalous physiological response. In the controls, the autofluorescence spectrum is characterized by an overall lower intensity, with two peaks having similar intensities centred at 490 nm and 540 nm. These peaks are mainly attributable to the fluorescence of collagen and elastin, which are key components of the skin extracellular matrix and which reflect a healthy skin condition, without significant inflammations.

In contrast, the autofluorescence profiles in the subjects with skin pathologies show marked differences. These spectra are characterized by a prominent peak centred at 490 nm, accompanied by a lower emission at 460 nm. The presence of an accentuated peak at 490 nm can be associated to the increase in NADH (nicotinamide adenine dinucleotide) and FAD (flavin adenine dinucleotide), two coenzymes involved in the cell metabolic processes which increase under conditions of oxidative stress and inflammation. The appearance of an emission at 460 nm, even if less intense, can show the presence of advanced glycation products (AGE), which are known to increase under pathological and inflammatory conditions of the skin.

Having a more numerous pool of patients in case of the psoriasis pathology (n=12), this condition was analysed more in detail, by concentrating on the not photo-exposed (arm) and pathologically active area, the results thereof are shown in Figures 19 to 22.

Even in this case, the phasor plots and respective enlargement (Figures 19 and 20) were reported, where the areas are clearly distinguishable. The not photo-exposed area concentrates in the graph portion in which the values of G are negative and the values of S are positive whereas the area where the pathology is active positions below the first one.

In the analysis of the mean spectral profiles of the subjects with psoriasis, significant differences between the examined areas in terms of intensity are observed: the not photo-exposed area shows the highest intensity values, whereas the pathological one has lower values. In the pathology active area, the lower intensity values suggest an increase in the cell metabolism and an inflammatory response. Here, the activation of biological processes, such as angiogenesis and the high cell turnover, can lead to an increase in the concentration of fluorophores, although not sufficiently high to overcome the values of the not photo-exposed area. This behaviour is coherent with the idea that the inflamed areas could accumulate metabolites and fluorophores such as NADH and lipofuscin, which are known to show an increase in metabolism and cell stress. At last, the not photo-exposed area, by showing the highest intensity values, shows an accumulation of fluorophores, by signalling a high metabolic activity and inflammatory processes which characterize psoriasis. These observations are supported by the obtained statistical results which confirm the significance of the differences among the three areas, by highlighting the impact of psoriasis and exposure to the light on the cell and metabolic responses (Figure 22).

The analysis of the autofluorescence spectra, then, not only distinguishes clearly between healthy controls and subjects affected by skin pathologies, but it also allows to identify specific fluorophores associated to different pathological conditions. These results highlight the potential of the invention system as diagnostic tool for the skin inflammatory diseases, by providing a detailed vision of the underlying biochemical variations.

A preferred variant of the protocol for acquiring data for calibrating a system according to the present invention is illustrated hereinafter.

As first step, one proceeds with verifying the connection of all devices and components included in the system, already described above. The activation of the tool takes place together with the turning-on of a computer connected to the processing unit and to the opening of a specific programme. The system, then, is turned-on by a potentiometer, which is adjusted to set the intensity of light emission. On the display or on the screen, the formation of a spectrum is observed.

Then, the integration time is adjusted, which represents the time range within which a specific pulse from the sensory devices is recorded. A longer integration time allows a greater capturing of the radiations emitted by the skin, by increasing the area of the spectrum proportional to the integration time. Generally an integration time of 1000 ms is preferred to obtain results which can be expected in a short time. The selection of the emission power and of the integration time guarantee the absence of any harmful or potentially dangerous effect on the skin. The instrument emission peak preferably is 385 nm, in the UVA field, by allowing to reach derma.

The ray emitted by the LED source of the excitation unit crosses the bifurcated optical fibre as far as reaching the handpiece, positioned on the skin surface. The intensity of the different wavelengths of the collected autofluorescent light is measured through a spectrophotometer, which allows to construct a detailed emission spectrum.

The collected spectrum is analysed, thanks to the algorithm integrated in the microcontroller, with the phasor method, which allows to decompose the autofluorescence signals in cosinusoidal (G) and sinusoidal (S) components, in particular according to the relationships:

$$G = \frac{\sum_\lambda I(\lambda) \cos\left(\frac{2n\pi(\lambda_i - \lambda_0)}{L}\right)}{\sum_\lambda I(\lambda)}$$

$$S = \frac{\sum_\lambda I(\lambda) \sin\left(\frac{2n\pi(\lambda_i - \lambda_0)}{L}\right)}{\sum_\lambda I(\lambda)}$$

with $L = \lambda_f - \lambda_0$

In these formulas:

  ○ $I(\lambda)$ represents the signal intensity at the wavelength $\lambda$;
  ○ $n$ is the harmonic number;
  ○ $\lambda_i$ is the i-th wavelength;
  ○ $\lambda_0$ is the reference wavelength;
  ○ L is the spectral width in the wavelength range.

The application of the phasor method returns as result for each analysed spectrum the values G and S which are used to quantify the differences and represented as a point on the phasor plane, which is shown on video to the user. The mapping of the phasor plane allows to associate immediately the position of the point in the plane under inflammatory dermatological conditions, apart from applying clustering techniques to characterize different conditions linked to the health status of the skin. Moreover, it is detected that the system proves to be very effective in monitoring over time possible dermatological treatments, as shown in the cosmetological application.

The present invention has been described sofar by referring to preferred embodiments. It is to be meant that other embodiments belonging to the same inventive core may exist, all comprised within the protective scope of the herebelow reported claims.

**Claims**

1. A system (10) for diagnosing the health status of a patient's skin (P), comprising:

   - an excitation unit (30), comprising a light source (3) configured to emit an excitation light radiation;
   - a processing unit (20), comprising a spectrophotometer (4) and a microcontroller (2);
   - an optical fibre (5), comprising:

     a free distal terminal end (6), and
     a bifurcated proximal end (7), which includes a first end (71) arranged at said light source (3) and a second end (72) connected to said processing unit (20),

   - a handpiece (1) comprising a pass-through seat (16) configured to house said optical fibre (5);

   wherein said optical fibre (5) is configured:

     by means of said first end (71), to convey the excitation light radiation produced by said light source (3), and to emit it by means of said distal terminal end (6) at a portion of the patient's skin (P), and
     by means of said distal terminal end (6), to collect the autofluorescence light radiation emitted by the irradiated portion of skin (P) and by means of said second end (72) to convey it to said processing unit (20);
     wherein said spectrophotometer (4) is configured to determine the electromagnetic spectrum of the autofluorescence light radiation and said microcontroller (2) is configured to process all spectral components of the electromagnetic spectrum as determined by the phasor method, by decomposing them into cosinusoidal (G) and sinusoidal (S) components and by representing them on the Gauss plane,
     said microcontroller (2) being further configured so as to map the Gauss plane and to associate the position of the cosinusoildal (G) and sinusoidal (S) components represented therein with a biomarker and/or an inflammatory dermatological condition, according to association criteria stored in said microcontroller (2).

2. The system (10) according to claim 1, wherein said microcontroller (2) is configured to quantify the intensity of a plurality of wavelengths included in the autofluorescence electromagnetic spectrum and associated to a plurality of biomarkers, including at least one of the following: AGE, collagen, elastin, NADH.

3. The system (10) according to claim 1 or 2, wherein said microcontroller (2) comprises a screen (21) for displaying the Gauss plane on which the aforementioned components of the autofluorescence electromagnetic spectrum are represented.

4. The system (10) according to any one of the preceding claims, wherein said microcontroller (2) is configured to store the data on the components of the autofluorescence electromagnetic spectrum.

5. The system (10) according to any one of the preceding claims, wherein said handpiece (1) comprises a proximal body (12), a distal body (13) and a central body (14) interposed between said proximal body (12) and distal body (13), each one of said proximal, distal and central bodies (12, 13, 14) having a respective passage span (112, 113, 114) configured to house said optical fibre (5), wherein said proximal, distal and central bodies (12, 13, 14) are configured to be arranged in series such that the respective passage spans (112, 113, 114) are aligned with one another.

6. The system (10) according to any one of the preceding claims, wherein said handpiece (1) comprises flexible connecting elements (8) arranged to connect said central body (14) with said proximal body (12) and said distal body (13), respectively, by allowing the mutual movement thereof.

7. The system (10) according to any one of the preceding claims, wherein said pass-through seat (16) is arranged centrally with respect to each one of said proximal, distal and central bodies (12, 13, 14).

8. The system (10) according to any one of the preceding claims, wherein said central body (14) has a spherical shape.

9. The system (10) according to any one of the preceding claims, wherein said distal body (13) comprises a free terminal end designed to face the portion of the patient's skin (P), thereat a centring element (91), having a through-hole (90) for positioning said optical fibre (5), and a protection element (92), suitable for closing said through-hole (90) and transparent to excitation light radiation and autofluorescence light radiation, are arranged.

10. The system (10) according to any one of the preceding claims, wherein said excitation unit (30) comprises a device (31) for controlling the output of the excitation light emitted by said light source (3).

11. The system (10) according to any one of the preceding claims, wherein said light source (3) comprises one or more LEDs.

12. A method to determine the pathological or physiological condition of the patient's skin (P), comprising the following steps:

collecting an autofluorescence light radiation, emitted by a portion of the patient's skin (P) irradiated by means of an excitation light radiation;

determining the electromagnetic spectrum of the autofluorescence light radiation;

processing all spectral components of the electromagnetic spectrum as determined by the phasor method, by decomposing them in cosinusoidal (G) and sinusoidal (S) components and representing them on the Gauss plane;

mapping the Gauss plane and associating the position of the cosinusoidal (G) and sinusoidal (S) components represented therein with concentration and/or distribution values of a respective biomarker;

determining a physiological condition or a pathological condition of the patient's skin (P) depending on the type of biomarker and its concentration and/or distribution.

**FIG. 1**

**FIG. 2**

**FIG. 3**

FIG. 4

FIG. 5

FIG. 6

FIG. 7

15

**FIG. 8**

**FIG. 9**

**FIG. 10**

**FIG. 11**

**FIG. 12**

FIG. 13

FIG. 14

FIG. 15

FIG. 16

FIG. 17

FIG. 18

**FIG. 19**

**FIG. 20**

**FIG. 21**

Mean value

**FIG. 22**

Europäisches
Patentamt

European
Patent Office

Office européen
des brevets

**EUROPEAN SEARCH REPORT**

**Application Number**

EP 25 22 0076

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LAGARTO JOÃO L. ET AL: "Multispectral Depth-Resolved Fluorescence Lifetime Spectroscopy Using SPAD Array Detectors and Fiber Probes", SENSORS, vol. 19, no. 12, 13 June 2019 (2019-06-13), page 2678, XP093276020, CH ISSN: 1424-8220, DOI: 10.3390/s19122678 * page 2, paragraph 4; page 3, paragraph 2; page 4, paragraph 3; page 8, paragraph 2; figures 1,3,4 * | 1-12 | INV. A61B5/00 |
| A | SAITO NOGUEIRA MARCELO ET AL: "Portable fluorescence lifetime spectroscopy system for in-situ interrogation of biological tissues", JOURNAL OF BIOMEDICAL OPTICS, vol. 22, no. 12, 19 October 2017 (2017-10-19), page 1, XP093275944, 1000 20th St. Bellingham WA 98225-6705 USA ISSN: 1083-3668, DOI: 10.1117/1.JBO.22.12.121608 * abstract; figures 1,2,3 * | 1-12 | |
| | -----<br>-/-- | | TECHNICAL FIELDS SEARCHED (IPC)<br><br>A61B<br>G01J |

The present search report has been drawn up for all claims

| Place of search | Date of completion of the search | Examiner |
|---|---|---|
| The Hague | 27 January 2026 | Chacon Caldera, J |

EPO FORM 1503 03.82 (P04C01)

page 1 of 2

Europäisches
Patentamt

European
Patent Office

Office européen
des brevets

## EUROPEAN SEARCH REPORT

**Application Number**

EP 25 22 0076

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | THOMPSON ALEX ET AL: "Hyperspectral fluorescence lifetime fibre probe spectroscopy for use in the study and diagnosis of osteoarthritis and skin cancer", OPTICAL BIOPSY IX, SPIE, 1000 20TH ST. BELLINGHAM WA 98225-6705 USA, vol. 7895, no. 1, 10 February 2011 (2011-02-10), pages 1-10, XP060006875, DOI: 10.1117/12.875120 * abstract; figure 1 * | 1-12 | |
| A | US 2017/071509 A1 (PANDEY RISHIKESH [US] ET AL) 16 March 2017 (2017-03-16) * paragraphs [0044], [0047]; claims 2,3,17,34; figure 2 * | 1-12 | |
| A | US 2020/378830 A1 (GRATTON ENRICO [US] ET AL) 3 December 2020 (2020-12-03) * claims 1,14; figure 4 * | 1-12 | |
| A | WO 98/46133 A1 (LUI HARVEY [CA]; ZENG HAISHAN [CA] ET AL.) 22 October 1998 (1998-10-22) * claims 1,6,9,11,12; figures 1,3 * | 1-12 | TECHNICAL FIELDS SEARCHED (IPC) |

The present search report has been drawn up for all claims

| Place of search | Date of completion of the search | Examiner |
|---|---|---|
| The Hague | 27 January 2026 | Chacon Caldera, J |

EPO FORM 1503 03.82 (P04C01)

## ANNEX TO THE EUROPEAN SEARCH REPORT
## ON EUROPEAN PATENT APPLICATION NO.

EP 25 22 0076

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2026

| Patent document cited in search report | | Publication date | Patent family member(s) | | Publication date |
|---|---|---|---|---|---|
| US 2017071509 | A1 | 16-03-2017 | US | 2017071509 A1 | 16-03-2017 |
| | | | WO | 2017049000 A1 | 23-03-2017 |
| US 2020378830 | A1 | 03-12-2020 | US | 2020378830 A1 | 03-12-2020 |
| | | | US | 2022236109 A1 | 28-07-2022 |
| WO 9846133 | A1 | 22-10-1998 | AU | 7021198 A | 11-11-1998 |
| | | | US | 6008889 A | 28-12-1999 |
| | | | US | 6069689 A | 30-05-2000 |
| | | | WO | 9846133 A1 | 22-10-1998 |

EPO FORM P0459

For more details about this annex : see Official Journal of the European Patent Office, No. 12/82